# EUROPEAN PATENT SPECIFICATION

(11) **EP 4 358 094 B1**
(45) Date of publication and mention of the grant of the patent: **27.05.2026**
(21) Application number: 22936709.9
(22) Date of filing: 07.10.2022
(51) Int. Cl.: G16H 40/67, G16H 80/00, G16H 20/40, A61B 5/16, A61B 5/00, G16H 10/20, G16H 30/40

(54) **SYSTEM FOR REDESIGNING TREATMENT PRESCRIPTIONS ACCORDING TO EVALUATION OF TREATMENT EFFECTIVENESS**
SYSTEM ZUR NEUGESTALTUNG VON BEHANDLUNGSVORSCHRIFTEN ENTSPRECHEND DER BEWERTUNG DER BEHANDLUNGSWIRKSAMKEIT
SYSTÈME DE REDÉFINITION DES ORDONNANCES DE TRAITEMENT SELON UNE ÉVALUATION DE L'EFFICACITÉ DU TRAITEMENT

(30) Priority: 31.08.2022 KR 20220109809
(43) Date of publication of application: 24.04.2024
(73) Proprietor: Neurophet Inc., Seoul 06234 (KR)
(72) Inventor: KIM, Dong Hyeon, Namyangju-si Gyeonggi-do 12235 (KR); KIM, Tae Yeong, Yongin-si Gyeonggi-do 16846 (KR); YOON, Hyung Seuk, Seoul 06270 (KR); HAN, Sang Jin, Seoul 08784 (KR)
(74) Representative: ABG Intellectual Property Law, S.L.
(86) International application number: PCT/KR2022/015111
(87) International publication number: WO 2024/048836

(56) References cited:
- KR-A- 20110 088 233
- KR-A- 20180 126 911
- KR-A- 20190 026 640
- KR-A- 20210 014 085
- KR-B1- 101 564 542
- US-A1- 2018 104 484
- US-A1- 2020 402 674
- US-A1- 2022 016 413
- US-B1- 8 224 667

## Description

### FIELD OF THE INVENTION

The present invention relates to a system for redesigning a treatment prescription, and more particularly, to a system for redesigning treatment prescriptions according to evaluation of treatment effectiveness that evaluates treatment effectiveness according to prescriptions and redesign treatment prescriptions according to the evaluation of treatment effectiveness.

### BACKGROUND ART

Recently, as interest in remote medical treatment has increased, technologies for issuing prescriptions through remote medical treatment and supporting remote medical treatment in which medicines are taken or treatment is performed according to the corresponding prescriptions have been actively developed. However, since a legal and institutional system for performing the remote medical treatment has not yet been established, there are limitations in implementing full-scale remote medical treatment. As a preceding step for the implementation of full-scale remote medical treatment, attempts are being made to improve the efficiency of diagnosis or treatment by allowing patients to directly use a diagnostic device or treatment device rented from medical institutions at home to perform diagnosis or treatment and to provide feedback information on this to the medical institutions.

As a representative example, there is a case where patients rent a portable electrocardiogram device from a hospital, operate the electrocardiogram device at home to perform electrocardiogram measurement, and visit the hospital to provide a measurement result to medical staff to utilize the measurement result for diagnosis of arrhythmia, etc.

Meanwhile, the applicant of this application is developing a technology that applies this method to the treatment device such as a brain stimulation device.

The brain is an internal organ of the human head and is the highest central organ of the nervous system, and is divided into the cerebrum, cerebellum, midbrain, leg brain, and medulla oblongata. In addition, the brain generates a brain wave, which is a signal in which the sum of neuron activity levels is measured in the epidermis of the brain.

As a method of measuring a state of the brain, there are an electroencephalogram (EEG) test in which a pad equipped with electrodes is first mounted on the scalp to measure and examine brain waves received from the electrodes, a CT examination in which the brain is scanned by tomography from various angles using radiation or ultrasound, an MRI examination in which the brain is photographed by magnetic resonance, etc.

Various concepts are known in the field of neural stimulation of brain structures, and brain stimulation techniques for achieving a predetermined purpose by stimulating the brain are largely classified into an invasive brain stimulation technique and a non-invasive brain stimulation technique.

The invasive brain stimulation technique is a method of surgically infiltrating electrodes into the brain and applying electrical signals, and the non-invasive brain stimulation technique is a method of achieving a predetermined effect by stimulating the brain without invading electrodes into the skull.

Specific brain stimulation techniques include a deep electrical stimulation method, a transcranial magnetic stimulation (TMS) method, a transcranial electrical stimulation (TES) method, a transcranial direct current stimulation (tDCS) method, a transcranial random noise stimulation (tRNS) method, etc.

Among these brain stimulation techniques, the brain electrical stimulation technique using the transcranial direct current stimulation (tDCS) method is one of the relatively simple non-invasive brain stimulation techniques, and is known to be effective in cognitive ability improvement, and treating various cranial nerve diseases such as depression, attention deficit hyperactivity disorder (ADHD), epilepsy, dementia, and sleep disorders, and thus many related studies are being actively conducted.

In a method of stimulating the brain using a transcranial direct current stimulation (tDCS) device, when an anode and a cathode are connected to the transcranial direct current stimulation (tDCS) device that generates a direct current and current is injected into the anode electrode, the current passes through the brain and then returns to the cathode. In this case, the treatment of brain disease is performed by stimulating the brain while the current flows from the anode to the cathode.

However, in the case of using such a transcranial direct current stimulation device, various treatment plan settings, such as the strength of current stimulation, the cycle of applying current, the time of applying current stimulation for each cycle, how many times a week to perform current stimulation treatment, etc., are required, and treatment effectiveness appear differently depending on the treatment plan.

In addition, no matter how effective the treatment plan is, it cannot be effective if the patient does not faithfully perform the treatment according to the treatment plan. Since each patient feels different pain from the current stimulation, if the pain felt by the patient is excessive, problems in which the patient does not faithfully perform the treatment itself occur.

Therefore, in the case of treatment prescriptions using such a treatment device, it is necessary to closely observe the treatment effectiveness and patient's treatment compliance to determine the treatment effectiveness and adaptively modify the treatment plan.
Document US2022/016413A1 describes systems and methods for providing curated neurostimulation such that users are enabled to provide improved therapy in a home environment.
Document US2020/402674A1 describes a system and a method for modulating delivery of remote therapy to a patient having an implantable medical device (IMD).
Document US2018/104484A1 describes a system and method for PEMF tissue engineering enhances musculoskeletal tissue stimulation by monitoring treatment for compliance with treatment regimens.
Document US8224667B1 describes a therapy adherence system comprising at least one computer system, a database, and an application that, when executed on the at least one computer system, receives a message from a home-based patient device containing medication compliance information and physical condition information.

### SUMMARY OF THE INVENTION

A problem to be solved by the present invention is to provide a system for redesigning treatment prescriptions that evaluates a treatment effectiveness by comprehensively considering patient's treatment compliance, a patient's functional status, a patient's discomfort, etc. and redesigns the treatment prescriptions according to the treatment effectiveness when the patient performs treatment directly using the treatment device according to the treatment prescription.

In accordance with an unclaimed aspect of the present disclosure, there is provided a method for redesigning treatment prescriptions according to evaluation of treatment effectiveness performed in a system for redesigning treatment prescriptions according to a preferred embodiment of the present invention for solving the problems described above, the method including the steps of: (a) providing patient information and image information of a patient to a medical staff terminal and receiving treatment prescriptions from the medical staff terminal; (c) receiving a treatment image obtained by photographing a treatment appearance while the patient performing treatment according to the treatment prescriptions using a treatment device and treatment history information from a patient terminal; (d) transmitting a questionnaire related to the treatment to the patient terminal, receiving a questionnaire reply from the patient terminal, and transmitting the questionnaire reply and compliance calculated according to the treatment history information to a medical staff terminal; and (e) when the medical staff terminal outputs the questionnaire reply and the compliance to medical staff, and the medical staff determines the treatment effectiveness according to the questionnaire reply and the compliance, redesigns the treatment prescriptions, and transmits second treatment prescriptions, receiving the second treatment prescriptions, and performing again from the step (c) according to the second treatment prescriptions.

In addition, the method for redesigning treatment prescriptions may further include, before the step (a), a step of constructing a big data DB by collecting basic information related to the treatment performed using the treatment device, and generating and storing big data by structuring the basic information, in which the big data may be provided to the medical staff terminal for reference when the medical staff inputs the treatment prescriptions into the medical staff terminal.

In addition, the basic information may include at least one of personal information about patients (including at least one of age, gender, medical history, and type of underlying disease), image information obtained by photographing patients' lesions, treatment prescription information, patients' treatment history according to the treatment prescription information and patients' questionnaire reply about a corresponding treatment process, and patients' cognitive/motor/emotional function evaluation results and calculated treatment effectiveness.

In addition, in the step (a), the system for redesigning treatment prescriptions may extract and analyze data about a patient having the same attributes (including at least one of age, sex, underlying disease, and physical characteristics) as the patient among a plurality of data stored in the big data DB, and provide a treatment method able to be provided to the patient as treatment assistance information to the medical staff terminal.

In addition, in the step (d), the system for redesigning treatment prescriptions may analyze the treatment image to check whether or not the patient has performed treatment using the treatment device in an accurate way (treatment performance status) using image recognition technology, and if it is determined that the treatment has been properly performed, the system may calculate the compliance from the treatment history information, and then provide the questionnaire reply and the compliance to the medical staff terminal.

In addition, in the step (d), the system for redesigning treatment prescriptions may analyze replies to questions related to a motor function, a cognitive function, and an emotional function included in the questionnaire reply, evaluate the motor function, cognitive function, and emotional function, and then provide the function evaluation result to the medical staff terminal together with the questionnaire reply and the compliance.

In addition, another aspect of the present disclosure may further include, between the step (a) and the step (c), a step of (b) setting the treatment device by generating treatment device setting information according to the treatment prescriptions and transmitting the treatment device setting information to the treatment device connected in a wired manner or wirelessly, and, in step (e), the system for redesigning treatment prescriptions may proceed to step (b), generate treatment device setting information according to the second treatment prescriptions, and perform again from the step (b).

In addition, in the step (c), the patient terminal may be interlocked with the treatment device to receive the treatment history information stored in the treatment device from the treatment device when a treatment operation is completed, and transmit the treatment history information to the system for redesigning treatment prescriptions together with the treatment image.

In accordance with an embodiment of the present invention, there is provided a non-transitory computer readable storage medium according to the appended independent claim 5.

In accordance with still another embodiment of the present invention, there is provided a system for redesigning treatment prescriptions for solving the above technical problem described above includes a processor and a memory for storing predetermined instructions, and, in which the processor that has executed the instructions stored in the memory performs the steps of: (a) providing patient information and image information of a patient to a medical staff terminal and receiving treatment prescriptions from the medical staff terminal; (c) receiving a treatment image obtained by photographing a patient treatment appearance while the patient performing treatment according to the treatment prescriptions using a treatment device and treatment history information from a patient terminal; (d) transmitting a questionnaire related to treatment to the patient terminal, receiving a questionnaire reply from the patient terminal, and transmitting the questionnaire reply and compliance calculated according to the treatment history information to a medical staff terminal; and (e) when the medical staff terminal outputs the questionnaire reply and the compliance to the medical staff, and a medical staff determines the treatment effectiveness according to the questionnaire reply and the compliance, redesigns the treatment prescriptions, and transmits second treatment prescriptions, receiving the second treatment prescriptions, and performing again from step (c) according to the second treatment prescriptions.

In addition, the system for redesigning treatment prescriptions the method for redesigning treatment prescriptions, the processor further performs, before the step (a), a step of constructing a big data DB by collecting basic information related to treatment performed using the treatment device, and generating and storing big data by structuring the basic information, in which the big data may be provided to the medical staff terminal for reference when the medical staff inputs the treatment prescriptions into the medical staff terminal.

In addition, in preferred embodiments of the present invention, the basic information may include at least one of personal information about patients (including at least one of age, gender, medical history, and type of underlying disease), image information obtained by photographing patients' lesions, treatment prescription information, patients' treatment history according to the treatment prescription information and patients' questionnaire reply about a corresponding treatment process, and patients' cognitive/motor/emotional function evaluation results and calculated treatment effectiveness.

In addition, in the step (a) of the preferred embodiments of the present invention, the processor may extract and analyze data about a patient having the same attributes (including at least one of age, sex, underlying disease and physical characteristics) as the patient among a plurality of data stored in the big data DB, and provide a treatment method able to be provided to the patient as treatment assistance information to the medical staff terminal.

In addition, in the step (d) of the present invention, the processor analyzes the treatment image to check whether or not the patient has performed treatment using the treatment device in an accurate way (treatment performance status) using image recognition technology, and if it is determined that the treatment has been properly performed, the processor may calculate compliance from the treatment history information, and then provide the questionnaire reply and the compliance to the medical staff terminal.

In addition, in the step (d) of the present invention, the processor analyzes replies to questionnaires related to the motor function, cognitive function, and emotional function included in the questionnaire replies, evaluate the motor function, cognitive function, and emotional function, and then provide the function evaluation result to the medical staff terminal together with the questionnaire replies and the compliance.

In addition, according to the present invention, the processor further performs, between the step (a) and the step (c), a step of (b) setting the treatment device by generating treatment device setting information according to the treatment prescriptions and transmitting the treatment device setting information to the treatment device connected in a wired manner or wirelessly, and, in step (e), the processor may proceed to step (b), generate treatment device setting information according to the second treatment prescriptions, and perform the process again from step (b).

In addition, in the step (c) of the preferred embodiments of the present invention, the patient terminal may be interlocked with the treatment device to receive the treatment history information stored in the treatment device from the treatment device when a treatment operation is completed, and transmit the treatment history information to the system for redesigning treatment prescriptions together with the treatment image.

In the present invention, when the system for redesigning treatment prescriptions receives the treatment prescriptions from the medical staff terminal by providing patient information and image information of the patient's lesions to the medical staff terminal, sets the treatment device according to the treatment prescriptions, and delivers the treatment device to the patient, the patient performs treatment by himself/herself using the treatment device at home.

When the treatment is finished, the patient terminal transmits the treatment history information, the treatment image obtained by photographing the process of self-treatment, and the questionnaire replies to the system for redesigning treatment prescriptions. The system for redesigning treatment prescriptions calculates compliance from the treatment history information, evaluates the treatment motor/cognitive/emotional functions from the questionnaire reply, and then provides the compliance and the functional evaluation results to the medical staff terminal together with the questionnaire replies, and allows the medical staff to evaluate the effect of the treatment prescriptions. As a result of the evaluation, if it is determined that the existing treatment prescriptions are effective, the existing treatment prescriptions are maintained, but if it is determined that the existing treatment prescriptions is not effective, prescriptions are redesigned, and the treatment device is reset according to the redesigned treatment prescriptions and is delivered to the patient to be subjected to treatment again, thereby capable of evaluating the treatment effectiveness more rationally according to various grounds and providing the treatment prescriptions suitable for the patient.

### BRIEF DESCRIPTION OF THE DRAWINGS

The accompanying drawings are included to provide a further understanding of the present invention, and are incorporated in and constitute a part of this specification. The drawings illustrate exemplary embodiments of the present invention and, together with the description, serve to explain principles of the present invention. In the drawings:
FIG. 1 is a diagram illustrating the overall connection relationship of a system for redesigning treatment prescriptions according to evaluation of treatment effectiveness according to a preferred embodiment of the present invention;
FIG. 2 is a block diagram illustrating a detailed configuration of a system for redesigning treatment prescriptions according to evaluation of treatment effectiveness according to the preferred embodiment of the present invention; and
FIGS. 3A and 3B are flowcharts illustrating a method for redesigning treatment prescriptions according to evaluation of treatment effectiveness according to the present disclosure.

### DETAILED DESCRIPTION OF THE PREFERRED EMBODIMENTS

Hereinafter, preferred embodiments of the present invention will be described with reference to the drawings.

Here, the objects, features and advantages of the present invention described above will become more apparent through the following detailed description in conjunction with the accompanying drawings. However, various changes can be made to the present invention and the present invention can have various embodiments. Hereinafter, specific embodiments thereof will be illustrated in the drawings and described in detail.

The same reference numbers throughout the specification represent the same components in principle. In addition, components having the same function within the scope of the same idea appearing in the drawings of each embodiment are described using the same reference numerals.

In the entire specification, when a certain part "includes" a certain component, it means that the certain part may further include other components without excluding other components unless otherwise stated. In addition, terms such as "...unit" and "module" described in the specification mean a unit that processes at least one function or operation, which may be implemented as hardware or software or a combination of hardware and software.

If it is determined that a detailed description of a known function or configuration related to the present invention may unnecessarily obscure the gist of the present invention, the detailed description thereof will be omitted. In addition, numbers (e.g., first, second, etc.) used in the description process of this specification are only identification symbols for distinguishing one component from another component.

According to the present invention, a transcranial stimulation device is described as a treatment device 400. Other unclaimed embodiments are not limited thereto, and can be applied to all treatment devices 400 that a user can operate at home.

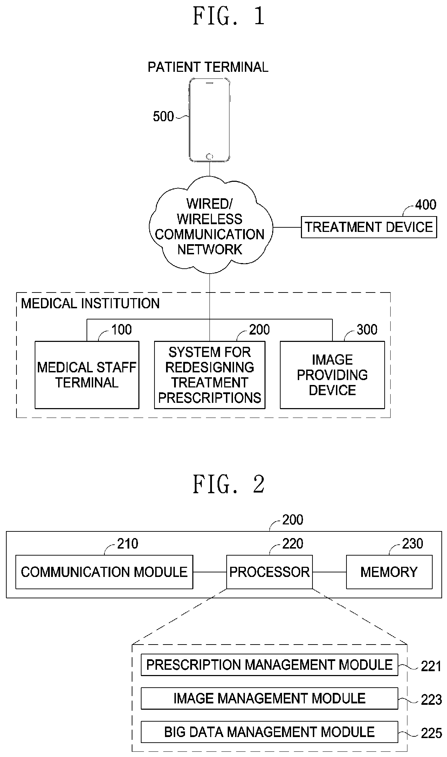

FIG. 1 is a diagram illustrating the overall connection relationship of a system for redesigning treatment prescriptions according to evaluation of treatment effectiveness 200 according to a preferred embodiment of the present invention.

Referring to FIG. 1, the system for redesigning treatment prescriptions according to evaluation of treatment effectiveness 200 according to a preferred embodiment of the present invention (hereinafter, abbreviated as "the system for redesigning treatment prescriptions 200") may be installed in a medical institution and connected to a medical staff terminal 100 and an image providing device 300 through an internal communication network of the medical institution.

In addition, the system for redesigning treatment prescriptions 200 may be connected to the treatment device 400 through a wired/wireless communication network or the internal communication network of the medical institution, and may be directly connected to the treatment device 400 in a wired manner according to a serial communication protocol such as USB. When the system for redesigning treatment prescriptions 200 is connected to the treatment device 400, it may transmit setting information to the treatment device 400 and receive and store treatment history information stored in the treatment device 400.

In addition, the system for redesigning treatment prescriptions 200 is connected to a patient terminal 500 through a wired/wireless communication network. When treatment is completed, the system for redesigning treatment prescriptions 200 may transmit a questionnaire about treatment to the patient terminal 500 and receive and store a reply to the questionnaire from the patient terminal 500.

The image providing device 300 connected to the system for redesigning treatment prescriptions 200 may be implemented as an image data acquisition device that photographs a patient's lesion and displays it as an image, such as a CT photographing device, an MRI photographing device, a PET photographing device, etc., or an image data storage device that collects and stores such image data in advance.

The medical staff terminal 100 receives patient information treatment history information, questionnaire information, and image information, etc. from the system for redesigning treatment prescriptions 200, outputs the information to medical staff, and transmits prescription information input by the medical staff to the system for redesigning treatment prescriptions 200 to issue prescriptions.

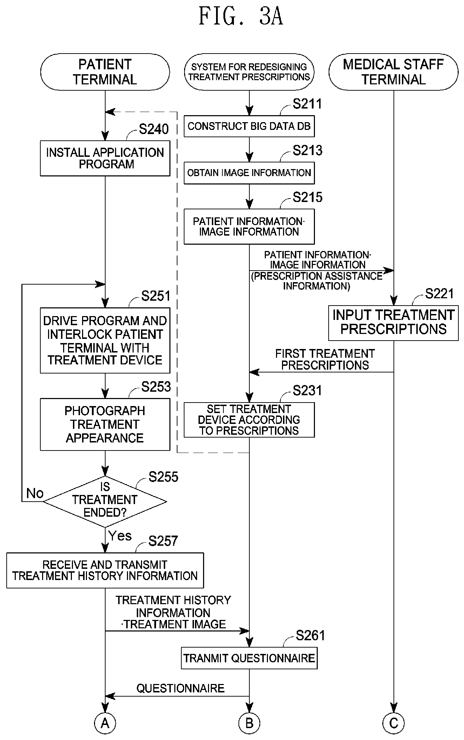

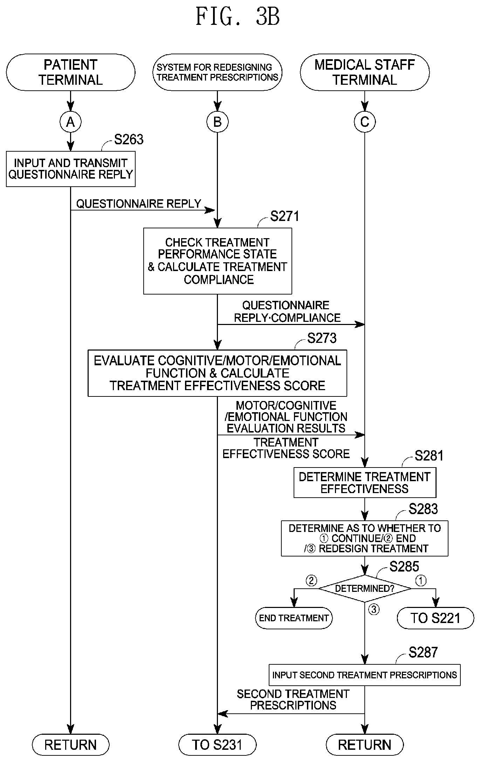

A more specific function of each of the components described above will be described later in more detail with reference to FIGS. 3A and 3B.

FIG. 2 is a block diagram illustrating the detailed configuration of the system for redesigning treatment prescriptions 200 according to evaluation of treatment effectiveness according to a preferred embodiment of the present invention.

Referring to FIG. 2, the system for redesigning treatment prescriptions 200 according to the preferred embodiment of the present invention includes a processor 220, a memory 230, and a communication module 210.

The memory 230 according to the preferred embodiment of the present invention may store instructions executable by the processor 220, programs executed by the processor 220, and input/output data in order to execute a method for redesigning treatment prescriptions according to evaluation of treatment effectiveness according to a preferred embodiment of the present invention.

In addition, the memory 230 stores an application program to be executed in the patient terminal 500 to perform the present invention, and stores questionnaire reply data and treatment history data received from the patient terminal 500. In addition, the memory 230 may store image information of patients received from the image providing device 300 and store prescription-related big data information collected in advance and structured.

The memory 230 may be implemented as a volatile memory such as a RAM(Random Access Memory), or may be implemented as a non-volatile memory such as a flash memory, ROM(Read-Only Memory), SSD(Solid State Drive) etc. The memory 230 may be operated by being replaced by web storage or cloud server that functions as a storage medium on the Internet.

The communication module 210 includes hardware configuration that enables communication with the medical staff terminal 100, image providing device 300, and treatment device 400. The communication module 210 can perform communication through wired or wireless methods. LAN (Local Area Network) or USB (Universal Serial Bus) communication is a typical example of the wired method, and other methods are possible. In the case of wireless methods, not only can WPAN (Wireless Personal Area Network) communication methods such as Bluetooth or Zigbee be used, but WLAN (Wireless Local Area Network) communication methods such as Wi-Fi or other known communication methods be used.

The processor 220 according to the preferred embodiment of the present invention can be implemented using a CPU (Central Processing Unit) or similar devices. The processor 220 controls the overall function of the system for redesigning treatment prescriptions 200 and executes instructions and programs stored in the memory 230 to perform each step of the method for redesigning treatment prescriptions described later with reference to FIGS. 3A to 3B.

In particular, when the processor 220 executes a program which is for executing the present invention and is stored in the memory 230, functions performed by the processor 220 may be functionally classified into a prescription management module, an image management module, and a big data management module.

Here, the prescription management module 221 performs steps related to issuing first treatment prescriptions and issuing second treatment prescriptions performed in the method for redesigning treatment prescriptions described later. The image management module 223 performs a function of storing patient's image information received from the image providing device 300 in the memory 230, and reading and managing the patient's image information. The big data management module 225 generates big data by synthesizing patient information, treatment prescription information, and image information and stores the big data in the memory 230, and provides big data information when the medical staff issues prescriptions, thereby performing a function of improving the efficiency of issuing treatment prescriptions.

In addition, the system for redesigning treatment prescriptions 200 may further include an input unit and an output unit, and the input unit (not illustrated) may be implemented as a typical input means such as a mouse and a keyboard, and receive setting information and selection information from an administrator and output the information to the processor 220. In addition, the output unit (not illustrated) may be implemented as a monitor, etc. to display data generated by the processor 220 to the administrator, etc.

FIGS. 3A and 3B are flowcharts illustrating the method for redesigning treatment prescriptions according to evaluation of treatment effectiveness according to the present disclosure.

Referring to FIGS. 3A and 3B, the method for redesigning treatment prescriptions according to evaluation of treatment effectiveness according to the present disclosure will be described.

First, the system for redesigning treatment prescriptions 200 collects basic information related to treatment performed using the treatment device 400 of the present invention in advance, generates big data by structuring the basic information, and stores the big data in the memory 230 to construct a big data DB (S211). The big data of the present invention can be generated by collecting information such as personal information about various patients (including age, gender, medical history, and type of underlying disease, etc.), image information obtained by photographing patients' lesions, first treatment prescription information, patients' treatment history according to the first treatment prescription information and patients' questionnaire replies about a corresponding treatment process, patients' cognitive/motor/emotional function evaluation results and calculated treatment effectiveness, redesigned second treatment prescription and patients' treatment history corresponding to the second treatment prescriptions and patients' questionnaire replies about a corresponding treatment process, cognitive/motor/emotional function evaluation results and calculated treatment effectiveness, and structuring these information, and a big data DB can be built by storing big data.

Here, as for a big data-based data collection and storage method, various technologies (e.g., big data collecting technology (log collection, crawling, sensing, RSS, Open API) and big data storing technology (NoSQL, File System, cloud, network)) are already known, and these various known technologies can be selectively applied according to circumstances. In this specification, a detailed description of the big data-based data collecting and storing method performed by the processor 220 is omitted.

For example, when the treatment device 400 of the present invention is the transcranial stimulation device, information about a plurality of patients, a plurality of brain images corresponding to each of the plurality of patients, a plurality of brain models corresponding to each of the plurality of brain images, a plurality of simulation results derived by simulating stimulation using each of the plurality of brain models, a plurality of prescription information generated based on the plurality of simulation results, and treatment effectiveness data according to the generated plurality of prescription information may be collected as big data basic information. And a big data DB may be implemented in the memory 230 by storing the collected data according to the big data-based data storing method.

Here, the big data described above is intended to provide useful reference information to the medical staff in issuing treatment prescriptions. In the case of an embodiment in which the medical staff does not utilize big data, the step S211 described above may be omitted.

Meanwhile, the system for redesigning treatment prescriptions 200 receives image information of the patient to whom the treatment prescriptions is to be issued from the image providing device 300 and stores the image information in the memory 230 (S213). As described above, the image providing device 300 may be a device that photographs patient's lesions in real time or a storage device that stores patient's image information photographed in advance.

Then, the system for redesigning treatment prescriptions 200 provides patient information and image information to the medical staff terminal 100 and requests treatment prescriptions (S215). The system for redesigning treatment prescriptions 200 requests a treatment prescription by providing patient information that basically includes the patient's basic personal information (including age, gender, medical history, type of underlying disease, etc.) and treatment history information stored in the memory 230, and the most recently captured image information together to the medical staff terminal 100.

In addition, if the big data DB is constructed in advance in step S211, the system for redesigning treatment prescriptions 200 may extract prescription assistance information useful for generating treatment prescriptions from the big data DB and provide the prescription assistance information together. That is, the system for redesigning treatment prescriptions 200 may extract and analyze data about the patient having the same attributes as the patient among the plurality of data stored as big data in the memory 230, and provide a list of treatment methods determined to be most effective as prescription assistance information.

For example, patients having the same attributes (e.g., age, gender, underlying disease, physical characteristics, etc.) as the patient may be selected from among a plurality of pre-stored data, data on the selected patients may be analyzed, and treatment methods that can be provided to patients to be subject to prescription issuance, setting information of the treatment device 400 for each treatment method, compliance and treatment effectiveness of the existing patients, etc. may be selected and provided to the medical staff terminal 100.

The medical staff terminal 100 outputs the patient information, image information, and treatment prescription assistance information received from the system for redesigning treatment prescriptions 200 to the medical staff, and receives the treatment prescriptions (referred to as first treatment prescriptions for distinction from redesigned second treatment prescriptions described later) from the medical staff and transmits the treatment prescriptions to the system for redesigning treatment prescriptions 200 (S221).

The system for redesigning treatment prescriptions 200 generates setting information (e.g., frequency of current pulse to be applied to the scalp, current intensity, duty ratio, total pulse application time, etc.) necessary for setting the operation of the treatment device 400 according to the treatment prescriptions (first treatment prescriptions) received from the medical staff terminal 100, and transmits the setting information to the treatment device 400 connected through a wired/wireless communication network or USB to set the treatment device 400 (S231). The setting information of the treatment device 400 means setting values necessary for performing an operation unique to the treatment device 400. When taking the transcranial stimulation device as an example, the setting information includes the period of stimulation pulses applied to each electrode, the maintenance length of each stimulation pulse (i.e., the duty ratio), the magnitude and intensity of each stimulation pulse, the total treatment time, etc. during performance of one treatment operation. In addition, the setting information of the treatment device 400 may include setting information prescribing how many times treatment is to be performed per day/week/month.

In this case, it is preferable that the treatment device 400 receives setting information and automatically sets settings according to the setting information, but, depending on the specifications of the treatment device 400, may be implemented to display setting information to a patient or an administrator, and allow the patient or the administrator to set settings using a button or the like equipped in the treatment device 400.

Meanwhile, the treatment device 400 that has been set is rented or transferred to the patient, and, prior to bringing the treatment device 400 home and operating it, the patient accesses the system for redesigning treatment prescriptions 200 using his/her mobile communication terminal (patient terminal 500), downloads and installs an application program for using the service of the present invention from the system for redesigning treatment prescriptions 200 (S240).

When the installation of the application program is completed, the user drives a program installed in the patient terminal 500, and the driven program interlocks the patient terminal 500 and the treatment device 400 with each other (S251). In a preferred embodiment of the present invention, the treatment device 400 and the patient terminal 500 are connected by having a Bluetooth communication module 210 therein and performing Bluetooth communication with each other, but, depending on the embodiment, the patient terminal 500 and the treatment device 400 may be connected in a wired manner through a USB communication cable.

When the patient terminal 500 and the treatment device 400 are interlocked with each other, the treatment device 400 performs a treatment operation according to a predefined set value, and the patient terminal 500 drives a camera to photograph a treatment process in which a user uses the treatment device 400 as a video or still image (S253).

When the treatment operation is completed in the treatment device 400 (S255), the treatment device 400 stores treatment history information therein, transmits the treatment history information to the patient terminal 500, and the patient terminal 500 that has received the treatment history information transmits a video or still image obtained by photographing the treatment process to the system for redesigning treatment prescriptions 200 together with the treatment history information (S257). In a preferred embodiment of the present invention, the treatment history information may include treatment execution date and time, treatment time, treatment completion status, treatment setting information, etc.

The system for redesigning treatment prescriptions 200 that has received the treatment image and treatment history information transmits a questionnaire for verifying treatment effectiveness to the patient terminal 500 (S261).

The questionnaire includes questions about changes in the user's condition or various inconveniences felt by the user in the process of performing treatment using the treatment device 400. For such a questionnaire survey, the questionnaire may be provided in a text format or a graphic UI format. For example, in the questionnaire, a specific selection menu for discomfort felt during the treatment process may be simply presented, and then the patient may be allowed to select a corresponding item from the menu.

In addition, in the questionnaire, questions and selection menus for allowing the patient to evaluate the cognitive function, motor function, and emotional function are presented and the patient may be allowed to input an option for the selection menus. For example, after completion of the treatment, the system for redesigning treatment prescriptions 200 outputs a text or voice guidance to the user, "Lift your arm as high as possible" as a type of questionnaire through the patient terminal 500, presents the selection menu displayed as a picture to the user, and allows the user to make a selection.

In the same way, the the system for redesigning treatment prescriptions 200 may proceed with a questionnaire for checking the patient's cognitive function by receiving today's date through the patient terminal 500, showing a specific picture and receiving an input of what this picture is, receiving input of words shown for a short time, listing words having commonalities and receiving the commonalities of the words, or enumerating several words, classifying common words, and receiving the common words through the patient terminal 500.

In addition, the system for redesigning treatment prescriptions 200 may make a query about the intensity of pain (e.g., pain intensity on a scale of 0 to 10) felt by the patient immediately after treatment or after a certain period of time has elapsed after treatment as a questionnaire, and receive the intensity of pain through the patient terminal 500 as a reply to the query.

In addition, the system for redesigning treatment prescriptions 200 may make a query whether or not adverse reactions such as redness, stinging and itching occurred during or after treatment as a questionnaire, and may obtain information about a case where the number of times, degree, and range of an abnormal reaction input as a reply to the query exceeds a predetermined value through the patient terminal 500.

As another example, the system for redesigning treatment prescriptions 200 may provide a questionnaire used for performing daily life evaluation (modified barthel index (MBI)), quality of life (SF-36) and self-efficacy evaluation according to pre-set criteria to the user through the patient terminal 500, and may obtain the evaluation result derived as the patient performs the above evaluation through the patient terminal 500.

The patient terminal 500 outputs the questionnaire described above to the user, and transmits an answer input with respect to the questionnaire by the user to the system for redesigning treatment prescriptions 200 as a questionnaire reply (S263).

The system for redesigning treatment prescriptions 200 analyzes the treatment image received from the patient terminal 500 and checks whether or not the patient uses the treatment device 400 in an accurate way (treatment execution state) using image recognition technology. If it is determined that the treatment has been properly performed, the system for redesigning treatment prescriptions 200 calculates treatment compliance from treatment history information, and then provides the questionnaire reply and compliance to the medical staff terminal 100 (S271).

When the user performs treatment at home using the treatment device 400, one of the important factors determining the treatment effectiveness is whether or not the user uses the treatment device 400 according to the correct usage. For example, in the case of the transcranial stimulation device, a user wears a stimulation device on the head and applies electrical stimulation to the head through electrodes. In order to accurately set the stimulation position, both ears must be exposed to the outside while the user is wearing the stimulation device. If a part of the ear is covered, since the stimulation device is not worn at the correct position, a position of the electrode is also out of an optimal position, which makes it difficult to obtain desired treatment effectiveness.

In addition, in an extreme case, by misunderstanding how to use the treatment device 400, the treatment device 400, which should be in contact with the chest, is used completely differently from the usage of the treatment device 400, such as contacting the stomach. In this case, determining the treatment effectiveness itself may be meaningless.

Accordingly, the system for redesigning treatment prescriptions 200 recognizes an object in the treatment image received from the patient terminal 500 and, when it is determined that the user has used the treatment device 400 in an accurate manner, evaluates treatment compliance, and performs a prescription redesign process to be described later.

The patient's treatment compliance may mean a value numerically indicating whether the patient accurately has performed treatment according to treatment prescription information. More specifically, the system for redesigning treatment prescriptions 200 may obtain a patient's treatment execution history according to the treatment prescription information from the patient terminal 500, and calculate the patient's treatment compliance with respect to the treatment prescription information (first treatment prescription information) based on the patient's treatment execution history.

For example, the system for redesigning treatment prescriptions 200 may set a basic score for the patient, and may add a score of a predetermined size whenever the patient properly performs treatment according to the treatment prescription information, or subtracts a score of a predetermined size whenever the patient fails to properly perform the treatment according to the treatment prescription information to calculate the patient's treatment compliance. The method of obtaining compliance is applied in various ways.

If the treatment prescriptions prescribe that treatment is performed once a day using the treatment device 400 every day for 30 days, and the time required for one treatment is 5 minutes, compliance can be calculated by setting 70 points as the basic score, adding +1 point each time the patient performs treatment once a day in the correct way, adding +0.5 points if the treatment time is stopped after 3 minutes, and -1 point is added for each omission of treatment once a day.

As an example, the system for redesigning treatment prescriptions 200 may calculate compliance for the number of treatments according to a formula: (Number of actual treatments performed/Number of treatments specified in prescriptions)* 100 and convert the corresponding score into a predefined score. When calculating the compliance, if the actual treatment time for one treatment is not more than 70% of the treatment time prescribed in the prescriptions, the number of treatments may not be added. In addition, when the total treatment time actually performed is less than the total treatment time prescribed in the prescriptions, a predefined score (e.g., 5 points) may be deducted.

As a specific example, in the case where the prescriptions are prescribed as (50 stimulations for 100 days/30 minutes per stimulation) and the actual treatment performed by the patient is (complete 45 stimulation performances for 100 days/perform 25 minutes once), if "(45/50)*100" is converted based 70 points, 63 points are calculated as compliance, and the total treatment time is insufficient, and thus, if 5 points are subtracted, the total compliance score is 58 points.

Meanwhile, according to another embodiment, the system for redesigning treatment prescriptions 200 may analyze the reply to the questions related to the motor function, the cognitive function, and the emotional function included in the questionnaire reply, evaluate the motor function, cognitive function, and emotional function, and then provides the function evaluation result to the medical staff terminal 100 together with the questionnaire reply and compliance, or may calculate treatment effectiveness and provide them to the medical staff terminal 100 (S273).

As an example of a function evaluation method, when comparing before and after treatment performance, if a reply to the questionnaire that motor function has improved is made, +10, if a reply to the questionnaire that motor function is the same is made, +0, and if a reply to the questionnaire that motor function is deteriorated is made, - 10 can be calculated as a motor function evaluation score.

Cognitive function is also evaluated in the same way. When comparing before and after treatment performance, if a reply to the questionnaire that cognitive function has improved is made, +10, if a reply to the questionnaire that cognitive function is the same is made, +0, and if a reply to the questionnaire that cognitive function is deteriorated is made, -10 can be calculated as a cognitive function evaluation score.

Also, in relation to the emotional function, when comparing before and after treatment performance, if a reply to the questionnaire that emotional function has improved is made, +10, if a reply to the questionnaire that emotional function is the same is made, +0, if a reply to the questionnaire that emotional function is deteriorated is made, -10 can be calculated as an emotional function evaluation score.

in addition, according to another embodiment, when a patient visits a medical institution and performs separate motor function and cognitive function tests, the administrator may directly input a corresponding test result into the system for redesigning treatment prescriptions 200, and the system for redesigning treatment prescriptions 200 may provide the test result to the medical staff terminal 100 together with the questionnaire reply.

In addition, in the step S273 described above, the system for redesigning treatment prescriptions 200 may calculate a treatment effectiveness score by analyzing the content included in the questionnaire reply described above and comprehensively considering the functional evaluation. For example, a final treatment effectiveness score may be calculated by summing up the compliance and functional evaluation scores described above.

In addition, in the process of redesigning the prescriptions described later, if the medical staff does not use the information on the evaluation results of cognitive function, motor function and cognitive function and the treatment effectiveness score, the cognitive/motor/emotional function evaluation through the questionnaire performed in step S273 may be omitted.

Meanwhile, the medical staff terminal 100 basically displays the questionnaire reply and compliance received from the system for redesigning treatment prescriptions 200 to the medical staff, and displays the motor function, cognitive function, and emotional function test results and the calculated treatment effectiveness to the medical staff as additional information, the medical staff determines the treatment effectiveness for the first treatment prescription based on these materials, and the medical staff terminal 100 receives the determination result (S281).

The medical staff terminal 100 receives an input from the medical staff as to whether to end treatment, continue treatment as the existing prescription, or redesign the treatment prescriptions and proceed with treatment according to the final determination result on the treatment effectiveness of the first treatment prescriptions (S283).

In step S283, the medical staff may make a decision by referring to the treatment effectiveness score calculated in step S273. If the combined score is calculated as 90 points or more, the medical staff may decide to maintain or terminate treatment, if the combined score exceeds 70 points and is less than 90 points, the medical staff may decide to redesign the prescriptions or end treatment, if the combined score is 50 or more to 70 or less, the medical staff may decide to maintain treatment, and if the combined score is less than 50, the medical staff may decide to redesign the prescriptions by changing the intensity/frequency/stimulation location of treatment.

In addition, when, in the state in which compliance is 70 points, the emotional function evaluation is adversely affected -10, and an aggravated case is found in the motor or functional evaluation, a decision to stop treatment may be made.

In step S283, if it is decided to end the treatment, the process of the present invention ends, and if it is decided to continue the treatment with the same prescriptions, the medical staff terminal 100 proceeds to the step S221 described above and reissues the same prescriptions (S285).

On the other hand, if the medical staff determines that it is necessary to redesign the treatment prescription by comprehensively considering the questionnaire reply, compliance, motor/cognitive/emotional function evaluation results, and treatment effectiveness score, the medical staff terminal 100 receives treatment prescriptions different from the first treatment prescriptions from the medical staff and transmits the treatment prescriptions to the system for redesigning treatment prescriptions 200 (S287).

In step S287, the medical staff terminal 100 may receive prescription assistance information useful for generating second treatment prescriptions from the prescription redesign device 200, similarly to step S221. That is, when the medical staff terminal 100 requests big data information from the system for redesigning treatment prescriptions 200, the system for redesigning treatment prescriptions 200 extracts and analyzes data about a patient having the same attributes as the patient among a plurality of data stored as big data in the memory 230 and provides the extracted data to the medical staff terminal 100.

For example, it is possible to provide a treatment method that can be provided to a patient to be subjected to prescriptions issuance by selecting patients having the same attributes (e.g., age, gender, underlying disease, and physical characteristics) as the patient among a plurality of pre-stored data and analyzing the data of the selected patients, and select setting information of the treatment device 400 for each treatment method, patient compliance, treatment effectiveness, etc., and provide them to the medical staff terminal 100.

The system for redesigning treatment prescriptions 200 that has received the second treatment prescriptions proceeds to step S231 and sets the treatment device 400 according to the second treatment prescriptions. In this case, the setting according to the second treatment prescriptions of the treatment device 400 may be performed according to the second treatment prescriptions by the administrator after the patient brings the treatment device 400 to a medical institution, and may be performed automatically by the system for redesigning treatment prescriptions 200 transmitting setting information according to the second treatment prescriptions to the patient terminal 500 and the patient terminal 500 transmitting the setting information to the treatment device 400 interlocked to the patient terminal 500.

The method for redesigning treatment prescriptions according to evaluation of treatment effectiveness described so far may be implemented as a computer program implemented as instructions executable on a computer and stored in a non-transitory storage medium.

The storage medium includes all kinds of recording devices in which data that can be read by a computer system is stored. Examples of computer-readable storage media include a ROM, RAM, CD-ROM, optical data storage device, etc. In addition, the computer-readable storage medium may be distributed in computer systems connected through a network, so that computer-readable codes can be stored and executed in a distributed manner.

So far, the present invention has been described focusing on preferred embodiments. Those skilled in the art to which the present invention pertains will understand that the present invention can be implemented in a modified form without departing from the essential characteristics of the present invention. Therefore, the disclosed embodiments should be considered from an explanatory point of view rather than a limiting point of view. The scope of the present invention is shown in the claims rather than the description described above.

## Claims

1. A system for redesigning treatment prescriptions (200) comprising:
a processor (220); and
a memory (230) that stores predetermined instructions, wherein
the processor (220) that has executed the instructions stored in the memory (230) performs the steps of:
(a) providing patient information and image information of a patient to a medical staff terminal (100) and receiving treatment prescriptions from the medical staff terminal (100);
(c) receiving a treatment image obtained by photographing a treatment appearance while the patient performing treatment according to the treatment prescriptions using a transcranial stimulation device (400) and treatment history information from a patient terminal (500);
(d) transmitting a questionnaire related to treatment to the patient terminal (500), receiving a questionnaire reply from the patient terminal (500), and transmitting the questionnaire reply and compliance calculated according to the treatment history information to a medical staff terminal (100); and
(e) when the medical staff terminal (100) outputs the questionnaire reply and the compliance to medical staff, and the medical staff determines the treatment effectiveness according to the questionnaire reply and the compliance, redesigns the treatment prescriptions, and transmits second treatment prescriptions, receiving the second treatment prescriptions from the medical staff terminal (100), and performing again from the step (c) according to the second treatment prescriptions;
wherein the processor (220) further performs, before the step (a), a step of constructing a big data DB by collecting basic information related to treatment performed using the transcranial stimulation device (400), and generating and storing big data by structuring the basic information,
wherein the big data is provided to the medical staff terminal (100) for reference when the medical staff inputs the treatment prescriptions into the medical staff terminal (100),
wherein in the step (d), the processor (220) analyzes the treatment image to check whether or not the patient has performed treatment using the transcranial stimulation device (400) in an accurate way using image recognition technology, and if it is determined that the treatment has been properly performed, the processor (220) calculates compliance from the treatment history information, and then provides the questionnaire reply and the compliance to the medical staff terminal (100),
wherein in the step (d), the processor (220) analyzes reply to questionnaires related to a motor function, a cognitive function, and an emotional function included in the questionnaire reply, evaluates the motor function, the cognitive function, and the emotional function, and then provides the function evaluation result to the medical staff terminal (100) together with the questionnaire reply and the compliance,
and wherein the processor (220) further performs, between the step (a) and the step (c), a step of (b) setting the transcranial stimulation device (400) by generating transcranial stimulation device (400) setting information according to the treatment prescriptions and transmitting the transcranial stimulation device (400) setting information to the transcranial stimulation device (400) connected in a wired manner or wirelessly, and, in the step (e), the processor (220) proceeds to step (b), generates transcranial stimulation device (400) setting information according to the second treatment prescriptions, and performs again from step (b) .

2. The system (200) of claim 1, wherein
the basic information includes at least one of personal information about patients including at least one of age, gender, medical history, and type of underlying disease, image information obtained by photographing patients' lesions, treatment prescription information, patients' treatment history according to the treatment prescription information, and patients' questionnaire reply about a corresponding treatment process, and patients' cognitive/motor/emotional function evaluation results and calculated treatment effectiveness.

3. The system (200) of claim 1, wherein
in the step (a), the processor (220) extracts and analyzes data about a patient having the same attributes including at least one of age, sex, underlying disease and physical characteristics as the patient among a plurality of data stored in the big data DB, and provides a treatment method able to be provided to the patient as treatment assistance information to the medical staff terminal (100).

4. The system (200) of claim 1, wherein
in the step (c), the patient terminal (500) is interlocked with the transcranial stimulation device (400) to receive the treatment history information stored in the transcranial stimulation device (400) from the transcranial stimulation device (400) when a treatment operation is completed, and transmits the treatment history information to the system for redesigning treatment prescriptions (200) together with the treatment image.

5. A non-transitory computer readable storage medium storing a computer program which, when executed by a computer including a processor (220), cause the processor (220) of the computer to carry out the following method for redesigning treatment prescriptions:
(a) providing patient information and image information of a patient to a medical staff terminal (100) and receiving treatment prescriptions from the medical staff terminal (100);
(c) receiving a treatment image obtained by photographing a treatment appearance while the patient performing treatment according to the treatment prescriptions using a transcranial stimulation device (400) and treatment history information from a patient terminal (500);
(d) transmitting a questionnaire related to treatment to the patient terminal (500), receiving a questionnaire reply from the patient terminal (500), and transmitting the questionnaire reply and compliance calculated according to the treatment history information to a medical staff terminal (100); and
(e) when the medical staff terminal (100) outputs the questionnaire reply and the compliance to medical staff, and the medical staff determines the treatment effectiveness according to the questionnaire reply and the compliance, redesigns the treatment prescriptions, and transmits second treatment prescriptions, receiving the second treatment prescriptions from the medical staff terminal (100), and performing again from the step (c) according to the second treatment prescriptions;
wherein the processor (220) further performs, before the step (a), a step of constructing a big data DB by collecting basic information related to treatment performed using the transcranial stimulation device (400), and generating and storing big data by structuring the basic information,
wherein the big data is provided to the medical staff terminal (100) for reference when the medical staff inputs the treatment prescriptions into the medical staff terminal (100),
wherein in the step (d), the processor (220) analyzes the treatment image to check whether or not the patient has performed treatment using the transcranial stimulation device (400) in an accurate way using image recognition technology, and if it is determined that the treatment has been properly performed, the processor (220) calculates compliance from the treatment history information, and then provides the questionnaire reply and the compliance to the medical staff terminal (100),
wherein in the step (d), the processor (220) analyzes reply to questionnaires related to a motor function, a cognitive function, and an emotional function included in the questionnaire reply, evaluates the motor function, the cognitive function, and the emotional function, and then provides the function evaluation result to the medical staff terminal (100) together with the questionnaire reply and the compliance,
and wherein the processor (220) further performs, between the step (a) and the step (c), a step of (b) setting the transcranial stimulation device (400) by generating transcranial stimulation device (400) setting information according to the treatment prescriptions and transmitting the transcranial stimulation device (400) setting information to the transcranial stimulation device (400) connected in a wired manner or wirelessly, and, in the step (e), the processor (220) proceeds to step (b), generates transcranial stimulation device (400) setting information according to the second treatment prescriptions, and performs again from step (b).

## Patentansprüche

1. Ein System zur Neugestaltung von Behandlungsvorschriften (200), umfassend:
einen Prozessor (220); und
einen Speicher (230), der vorbestimmte Befehle speichert, wobei
der Prozessor (220), der die in dem Speicher (230) gespeicherten Befehle ausgeführt hat, die folgenden Schritte durchführt:
(a) Übermitteln von Patienteninformationen und Bildinformationen eines Patienten zu einem Terminal des medizinischen Personals (100) und Empfangen von Behandlungsvorschriften von dem Terminal des medizinischen Personals (100);
(c) Empfangen eines durch Fotografieren eines Erscheinungsbildes der Behandlung gewonnenen Behandlungsbildes, während der Patient eine Behandlung gemäß den Behandlungsvorschriften unter Verwendung eines Geräts zur transkraniellen Stimulation (400) und von Informationen zur Behandlungsgeschichte aus einem Patiententerminal (500) erfährt;
(d) Übertragen eines Fragebogens zu der Behandlung zu dem Patiententerminal (500), Empfangen einer Antwort auf den Fragebogen von dem Patiententerminal (500), und Übertragen der Antwort auf den Fragebogen und der gemäß den Informationen zur Behandlungsgeschichte berechneten Compliance zu einem Terminal des medizinischen Personals (100); und
(e) wenn das Terminal des medizinischen Personals (100) die Antwort auf den Fragebogen und die Compliance an das medizinische Personal ausgibt, und das medizinische Personal die Wirksamkeit der Behandlung gemäß der Antwort auf den Fragebogen und der Compliance ermittelt, Neugestaltung der Behandlungsvorschriften und Übertragen der zweiten Behandlungsvorschriften, Empfangen der zweiten Behandlungsvorschriften von dem Terminal des medizinischen Personals (100) und erneutes Durchführen von Schritt (c) gemäß den zweiten Behandlungsvorschriften;
wobei der Prozessor (220) ferner, vor Schritt (a), einen Schritt des Aufbaus einer Big-Data-Datenbank durch Sammeln von Basisinformationen zu der unter Verwendung des Geräts zur transkraniellen Stimulation (400) durchgeführten Behandlung, und Erzeugen und Speichern von Big Data durch Strukturieren der Basisinformationen,
wobei die Big Data zu dem Terminal des medizinischen Personals (100) zu Informationszwecken übermittelt werden, wenn das medizinische Personal die Behandlungsvorschriften in das Terminal des medizinischen Personals (100) eingibt,
wobei in Schritt (d) der Prozessor (220) das Behandlungsbild analysiert, um zu prüfen, ob der Patient eine Behandlung unter Verwendung des Geräts zur transkraniellen Stimulation (400) auf eine genaue Weise mittels Bilderkennungstechnologie erfahren hat oder nicht, und wenn festgestellt wird, dass die Behandlung korrekt durchgeführt wurde, berechnet der Prozessor (220) die Compliance aus den Informationen zur Behandlungsgeschichte und übermittelt dann die Antwort auf den Fragebogen und die Compliance zu dem Terminal des medizinischen Personals (100),
wobei in Schritt (d) der Prozessor (220) die Antwort auf Fragebögen bezüglich einer motorischen Funktion, einer kognitiven Funktion und einer emotionalen Funktion, die in der Antwort auf den Fragebogen enthalten sind, analysiert, die motorische Funktion, die kognitive Funktion und die emotionale Funktion auswertet, und dann das Ergebnis der Funktionsauswertung zusammen mit der Antwort auf den Fragebogen und der Compliance zu dem Terminal des medizinischen Personals (100) übermittelt,
und wobei der Prozessor (220) ferner, zwischen Schritt (a) und Schritt (c), einen Schritt (b) durchführt, in dem das Gerät zur transkraniellen Stimulation (400) dadurch eingestellt wird, dass Einstellinformationen für das Gerät zur transkraniellen Stimulation (400) gemäß den Behandlungsvorschriften erzeugt werden und die Einstellinformationen des Geräts zur transkraniellen Stimulation (400) zu dem verdrahtet oder drahtlos angeschlossenen Gerät zur transkraniellen Stimulation (400) übertragen werden, und in Schritt (e) geht der Prozessor (220) weiter zu Schritt (b), erzeugt Einstellinformationen für das Gerät zur transkraniellen Stimulation (400) gemäß den zweiten Behandlungsvorschriften, und führt wieder Schritt (b) durch.

2. Das System (200) nach Anspruch 1, wobei
die Basisinformationen mindestens eines von persönlichen Informationen über Patienten einschließlich mindestens einem von Alter, Geschlecht, medizinischer Geschichte und Art der zugrundeliegenden Erkrankung, Bildinformationen, die durch Fotografieren von Läsionen der Patienten gewonnen wurden, Informationen zu den Behandlungsvorschriften, der Behandlungsgeschichte der Patienten gemäß den Informationen zu den Behandlungsvorschriften, und der Antwort auf den Fragebogen der Patienten zu einem entsprechenden Behandlungsverfahren, sowie der Ergebnisse der Auswertung der kognitiven/motorischen/emotionalen Funktion der Patienten und der berechneten Wirksamkeit der Behandlung beinhalten.

3. Das System (200) nach Anspruch 1, wobei
in Schritt (a) der Prozessor (220) Daten über einen Patienten mit denselben Attributen einschließlich mindestens einem von Alter, Geschlecht, zugrundeliegender Erkrankung und physikalischen Eigenschaften des Patienten unter einer Vielzahl von in der Big-Data-Datenbank gespeicherten Daten extrahiert und analysiert, und ein übermittelbares Behandlungsverfahren für den Patienten als Hilfsinformationen für die Behandlung zu dem Terminal des medizinischen Personals (100) übermittelt.

4. Das System (200) nach Anspruch 1, wobei
in Schritt (c) das Patiententerminal (500) mit dem Gerät zur transkraniellen Stimulation (400) verzahnt wird, um die in dem Gerät zur transkraniellen Stimulation (400) gespeicherten Informationen zur Behandlungsgeschichte von dem Gerät zur transkraniellen Stimulation (400) zu empfangen, wenn ein Behandlungsvorgang beendet ist, und die Informationen zur Behandlungsgeschichte zusammen mit dem Behandlungsbild zu dem System zur Neugestaltung von Behandlungsvorschriften (200) übertragen werden.

5. Ein nicht-transitorisches computerlesbares Speichermedium, auf dem ein Computerprogramm gespeichert ist, das bei Ausführung durch einen Computer mit einem Prozessor (220) den Prozessor (220) des Computers dazu veranlasst, das folgende Verfahren zur Neugestaltung von Behandlungsvorschriften auszuführen:
(a) Übermitteln von Patienteninformationen und Bildinformationen eines Patienten zu einem Terminal des medizinischen Personals (100) und Empfangen von Behandlungsvorschriften von dem Terminal des medizinischen Personals (100);
(c) Empfangen eines durch Fotografieren eines Erscheinungsbildes der Behandlung gewonnenen Behandlungsbildes, während der Patient eine Behandlung gemäß den Behandlungsvorschriften unter Verwendung eines Geräts zur transkraniellen Stimulation (400) und von Informationen zur Behandlungsgeschichte aus einem Patiententerminal (500) erfährt;
(d) Übertragen eines Fragebogens zu der Behandlung an das Patiententerminal (500), Empfangen einer Antwort auf den Fragebogen von dem Patiententerminal (500), und Übertragen der Antwort auf den Fragebogen und der gemäß den Informationen zur Behandlungsgeschichte berechneten Compliance zu einem Terminal des medizinischen Personals (100); und
(e) wenn das Terminal des medizinischen Personals (100) die Antwort auf den Fragebogen und die Compliance an das medizinische Personal ausgibt, und das medizinische Personal die Wirksamkeit der Behandlung gemäß der Antwort auf den Fragebogen und der Compliance ermittelt, Neugestaltung der Behandlungsvorschriften und Übertragen der zweiten Behandlungsvorschriften, Empfangen der zweiten Behandlungsvorschriften von dem Terminal des medizinischen Personals (100) und erneutes Durchführen von Schritt (c) gemäß den zweiten Behandlungsvorschriften;
wobei der Prozessor (220) ferner, vor Schritt (a), einen Schritt des Aufbaus einer Big-Data-Datenbank durch Sammeln von Basisinformationen zu der unter Verwendung des Geräts zur transkraniellen Stimulation (400) durchgeführten Behandlung, und Erzeugen und Speichern von Big Data durch Strukturieren der Basisinformationen,
wobei die Big Data zu dem Terminal des medizinischen Personals (100) zu Informationszwecken übermittelt werden, wenn das medizinische Personal die Behandlungsvorschriften in das Terminal des medizinischen Personals (100) eingibt,
wobei in Schritt (d) der Prozessor (220) das Behandlungsbild analysiert, um zu prüfen, ob der Patient eine Behandlung unter Verwendung des Geräts zur transkraniellen Stimulation (400) auf eine genaue Weise mittels Bilderkennungstechnologie erfahren hat oder nicht, und wenn festgestellt wird, dass die Behandlung korrekt durchgeführt wurde, berechnet der Prozessor (220) die Compliance aus den Informationen zur Behandlungsgeschichte und übermittelt dann die Antwort auf den Fragebogen und die Compliance zu dem Terminal des medizinischen Personals (100),
wobei in Schritt (d) der Prozessor (220) die Antwort auf Fragebögen bezüglich einer motorischen Funktion, einer kognitiven Funktion und einer emotionalen Funktion, die in der Antwort auf den Fragebogen enthalten sind, analysiert, die motorische Funktion, die kognitive Funktion und die emotionale Funktion auswertet, und dann das Ergebnis der Funktionsauswertung zusammen mit der Antwort auf den Fragebogen und der Compliance zu dem Terminal des medizinischen Personals (100) übermittelt,
und wobei der Prozessor (220) ferner, zwischen Schritt (a) und Schritt (c), einen Schritt (b) durchführt, in dem das Gerät zur transkraniellen Stimulation (400) dadurch eingestellt wird, dass Einstellinformationen für das Gerät zur transkraniellen Stimulation (400) gemäß den Behandlungsvorschriften erzeugt werden und die Einstellinformationen des Geräts zur transkraniellen Stimulation (400) zu dem verdrahtet oder drahtlos angeschlossenen Gerät zur transkraniellen Stimulation (400) übertragen werden, und in Schritt (e) geht der Prozessor (220) weiter zu Schritt (b), erzeugt Einstellinformationen für das Gerät zur transkraniellen Stimulation (400) gemäß den zweiten Behandlungsvorschriften, und führt wieder Schritt (b) durch.

## Revendications

1. Un système de redéfinition des prescriptions de traitement (200) comprenant :
un processeur (220) ; et
une mémoire (230) qui stocke des instructions prédéterminées, dans lequel
le processeur (220) qui a exécuté les instructions stockées dans la mémoire (230) effectue les étapes consistant à :
(a) fournir des informations sur le patient et des informations d'imagerie d'un patient à un terminal du personnel médical (100) et recevoir des prescriptions de traitement provenant du terminal du personnel médical (100) ;
(c) recevoir une image de traitement obtenue en photographiant l'aspect du traitement pendant que le patient suit le traitement conformément aux prescriptions de traitement à l'aide d'un dispositif de stimulation transcrânienne (400) ainsi que des informations d'historique de traitement provenant d'un terminal du patient (500) ;
(d) transmettre un questionnaire relatif au traitement au terminal du patient (500), recevoir une réponse au questionnaire provenant du terminal du patient (500), et transmettre la réponse au questionnaire et l'observance calculée en fonction des informations d'historique de traitement à un terminal du personnel médical (100) ; et
(e) lorsque le terminal du personnel médical (100) transmet la réponse au questionnaire et l'observance au personnel médical, et que le personnel médical détermine l'efficacité du traitement en fonction de la réponse au questionnaire et de l'observance, redéfinit les prescriptions de traitement et transmet des deuxièmes prescriptions de traitement, recevoir les deuxièmes prescriptions de traitement provenant du terminal du personnel médical (100), et recommencer à partir de l'étape (c) conformément aux deuxièmes prescriptions de traitement ;
dans lequel le processeur (220) exécute en outre, avant l'étape (a), une étape consistant à construire une base de données de mégadonnées en collectant des informations de base relatives au traitement effectué à l'aide du dispositif de stimulation transcrânienne (400), et générer et stocker des mégadonnées en structurant les informations de base,
dans lequel les mégadonnées sont fournies au terminal du personnel médical (100) à titre de référence lorsque le personnel médical saisit les prescriptions de traitement dans le terminal du personnel médical (100),
dans lequel, à l'étape (d), le processeur (220) analyse l'image de traitement afin de vérifier si le patient a effectué le traitement à l'aide du dispositif de stimulation transcrânienne (400) de manière précise en utilisant une technologie de reconnaissance d'images, et s'il est déterminé que le traitement a été correctement effectué, le processeur (220) calcule l'observance à partir des informations d'historique de traitement, puis fournit la réponse au questionnaire et l'observance au terminal du personnel médical (100),
dans lequel, à l'étape (d), le processeur (220) analyse les réponses aux questionnaires relatifs à une fonction motrice, une fonction cognitive et une fonction émotionnelle incluses dans la réponse au questionnaire, évalue la fonction motrice, la fonction cognitive et la fonction émotionnelle, puis fournit le résultat de l'évaluation des fonctions au terminal du personnel médical (100) avec la réponse au questionnaire et l'observance,
et dans lequel le processeur (220) effectue en outre, entre l'étape (a) et l'étape (c), une étape (b) consistant à configurer le dispositif de stimulation transcrânienne (400) en générant des informations de configuration du dispositif de stimulation transcrânienne (400) conformément aux prescriptions de traitement et en transmettant lesdites informations de configuration du dispositif de stimulation transcrânienne (400) au dispositif de stimulation transcrânienne (400) connecté de manière filaire ou sans fil, et, à l'étape (e), le processeur (220) passe à l'étape (b), génère des informations de configuration du dispositif de stimulation transcrânienne (400) conformément aux deuxièmes prescriptions de traitement, et recommence à partir de l'étape (b).

2. Le système (200) selon la revendication 1, dans lequel
les informations de base incluent au moins l'une des informations personnelles concernant les patients, y compris au moins l'un des éléments suivants : l'âge, le sexe, les antécédents médicaux et le type de maladie sous-jacente ; les informations d'imagerie obtenues en photographiant les lésions des patients ; les informations de prescription de traitement ; l'historique de traitement des patients selon les informations de prescription de traitement ; et les réponses des patients au questionnaire concernant un processus de traitement correspondant ; ainsi que les résultats d'évaluation des fonctions cognitives, motrices et émotionnelles des patients et l'efficacité du traitement calculée.

3. Le système (200) selon la revendication 1, dans lequel
à l'étape (a), le processeur (220) extrait et analyse des données concernant un patient présentant les mêmes attributs, incluant au moins l'un des éléments suivants : l'âge, le sexe, la maladie sous-jacente et les caractéristiques physiques du patient parmi une pluralité de données stockées dans la base de données de mégadonnées, et fournit une méthode de traitement pouvant être administrée au patient en tant qu'informations d'aide au traitement au terminal du personnel médical (100).

4. Le système (200) selon la revendication 1, dans lequel
à l'étape (c), le terminal du patient (500) est interconnecté avec le dispositif de stimulation transcrânienne (400) pour recevoir les informations d'historique de traitement stockées dans le dispositif de stimulation transcrânienne (400) à partir du dispositif de stimulation transcrânienne (400) lorsqu'une opération de traitement est terminée, et transmet les informations d'historique de traitement au système de redéfinition des prescriptions de traitement (200) conjointement avec l'image de traitement.

5. Un support de stockage non transitoire lisible par ordinateur, stockant un programme informatique qui, lorsqu'il est exécuté par un ordinateur incluant un processeur (220), amène le processeur (220) de l'ordinateur à mettre en œuvre le procédé suivant pour la redéfinition des prescriptions de traitement :
(a) fournir des informations sur le patient et des informations d'imagerie d'un patient à un terminal du personnel médical (100) et recevoir des prescriptions de traitement provenant du terminal du personnel médical (100) ;
(c) recevoir une image de traitement obtenue en photographiant l'aspect du traitement pendant que le patient suit le traitement conformément aux prescriptions de traitement à l'aide d'un dispositif de stimulation transcrânienne (400) et des informations d'historique de traitement provenant d'un terminal du patient (500) ;
(d) transmettre un questionnaire relatif au traitement au terminal du patient (500), recevoir une réponse au questionnaire provenant du terminal du patient (500), et transmettre la réponse au questionnaire et l'observance calculée en fonction des informations d'historique de traitement à un terminal du personnel médical (100) ; et
(e) lorsque le terminal du personnel médical (100) transmet la réponse au questionnaire et l'observance au personnel médical, et que le personnel médical détermine l'efficacité du traitement en fonction de la réponse au questionnaire et de l'observance, redéfinit les prescriptions de traitement et transmet des deuxièmes prescriptions de traitement, recevoir les deuxièmes prescriptions de traitement provenant du terminal du personnel médical (100), et recommencer à partir de l'étape (c) conformément aux deuxièmes prescriptions de traitement ;
dans lequel le processeur (220) exécute en outre, avant l'étape (a), une étape consistant à construire une base de données de mégadonnées en collectant des informations de base relatives au traitement effectué à l'aide du dispositif de stimulation transcrânienne (400), et générer et stocker des mégadonnées en structurant les informations de base,
dans lequel les mégadonnées sont fournies au terminal du personnel médical (100) à titre de référence lorsque le personnel médical saisit les prescriptions de traitement dans le terminal du personnel médical (100),
dans lequel, à l'étape (d), le processeur (220) analyse l'image de traitement afin de vérifier si le patient a effectué le traitement à l'aide du dispositif de stimulation transcrânienne (400) de manière précise en utilisant une technologie de reconnaissance d'images, et s'il est déterminé que le traitement a été correctement effectué, le processeur (220) calcule l'observance à partir des informations d'historique de traitement, puis fournit la réponse au questionnaire et l'observance au terminal du personnel médical (100),
dans lequel, à l'étape (d), le processeur (220) analyse les réponses aux questionnaires relatifs à une fonction motrice, une fonction cognitive et une fonction émotionnelle incluses dans la réponse au questionnaire, évalue la fonction motrice, la fonction cognitive et la fonction émotionnelle, puis fournit le résultat de l'évaluation des fonctions au terminal du personnel médical (100) avec la réponse au questionnaire et l'observance,
et dans lequel le processeur (220) effectue en outre, entre l'étape (a) et l'étape (c), une étape (b) consistant à configurer le dispositif de stimulation transcrânienne (400) en générant des informations de configuration du dispositif de stimulation transcrânienne (400) conformément aux prescriptions de traitement et en transmettant lesdites informations de configuration du dispositif de stimulation transcrânienne (400) au dispositif de stimulation transcrânienne (400) connecté de manière filaire ou sans fil, et, à l'étape (e), le processeur (220) passe à l'étape (b), génère des informations de configuration du dispositif de stimulation transcrânienne (400) conformément aux deuxièmes prescriptions de traitement, et recommence à partir de l'étape (b).
